# EUROPEAN PATENT SPECIFICATION

(11) **EP 2 389 966 B1**
(45) Date of publication and mention of the grant of the patent: **13.11.2013**
(21) Application number: 11177492.3
(22) Date of filing: 09.12.2009
(51) Int. Cl.: A61M 1/10, A61M 1/36, F04B 43/12

(54) **Peristaltic pump and blood processing apparatus with air bubble detector**
Peristaltische Pumpe und Blutverarbeitungsvorrichtung mit Luftblasendetektor
Pompe péristaltique et appareil de traitement du sang avec détecteur de bulles d'air

(30) Priority: 22.12.2008 US 139870 P
(43) Date of publication of application: 30.11.2011
(62) Divisional of application: 09795614.8
(73) Proprietor: Terumo BCT, Inc., Lakewood, CO 80215 (US)
(72) Inventor: Scibona, Joseph, Littleton, CO Colorado 80128 (US); Gibbons, David, Highlands Ranch, CO Colorado 80129 (US); Lindner, John, Morrison, CO Colorado 80465 (US); Palsulich, William, Lakewood, CO Colorado 80215 (US)
(74) Representative: Roberts, Mark Peter

(56) References cited:
- US-A- 5 533 877
- US-A- 5 941 842
- US-B1- 6 515 487

## Description

The present invention relates to an apparatus and method useful for separating particles or components of a biologic fluid, such as blood. The invention has particular advantages in connection with separating blood components, such as white blood cells and platelets.

In many different fields, liquids carrying particles must be filtered or processed to obtain either a purified liquid or purified particle end product. In its broadest sense, a filter is any device capable of removing or separating particles from a substance. Thus, the term "filter" as used herein is not limited to a porous media material but includes many different types of devices and processes where particles are either separated from one another or from liquid.

In the medical field, it is often necessary to filter blood. Whole blood consists of various liquid components and particle components. The liquid portion of blood is largely made up of plasma, and the particle components include red blood cells (erythrocytes), white blood cells (leukocytes), and platelets (thrombocytes). While these constituents have similar densities, their average density relationship, in order of decreasing density, is as follows: red blood cells, white blood cells, platelets, and plasma. In addition, the particle components are related according to size, in order of decreasing size, as follows: white blood cells, red blood cells, and platelets. Most current purification devices rely on density and size differences or surface chemistry characteristics to separate and/or filter the blood components.

Typically, donated platelets are separated or harvested from other blood components using a centrifuge. White cells or other selected components may also be harvested. The centrifuge rotates a blood separation vessel to separate components within the vessel or reservoir using centrifugal force. In use, blood enters the separation vessel while it is rotating at a very rapid speed and centrifugal force stratifies the blood components, so that particular components may be separately removed. Components are removed through ports arranged within stratified layers of blood components.

The invention is defined in the claims.

The present invention includes a centrifuge blood separation apparatus for separating particles suspended in a fluid, particularly blood and blood components. The apparatus may have a blood processing vessel mounted on a rotor of a centrifuge. In the process of removing blood from a donor, separating the blood into components, adding anticoagulant or replacement fluids, and returning blood components to a donor, air bubbles may be inadvertently introduced into the fluid. Small air bubbles are of little or no consequence, but large air bubbles in the returned blood components may be painful or harmful to the donor. It is desirable to monitor the returning blood components for air bubbles that are larger than a predetermined size. The apparatus of this invention provides a sensor in the outflow race of the return peristaltic pump. A sensor structure is uniquely mounted in an exit slot of the return peristaltic pump for detecting air bubbles in the fluid within a return loop.

The sensor structure comprises an inner protrusion which faces a similar outer protrusion, located in the exit slot. When a bag and tubing set is mounted on the blood processing device, a cassette is mechanically and automatically drawn into place on the device, which wedges a portion of the return loop between the protrusions. Outer surfaces of the return loop contact an appropriate sensor in the protrusions. Such a sensor is a sonic pulse echo sensor. A pre-determined minimum bubble size or sizes or a cumulative volume may be selected, and the device operator may be warned only of the existence of bubbles that exceed a certain size or of a cumulative volume of bubbles, or the blood donation procedure may be stopped if a bubble exceeds a certain critical size or if a pre-determined volume of bubbles over a certain period or volume of fluid is exceeded.

There is provided a centrifuge blood separation apparatus comprising a pump assembly adapted to interface with a tubing set, the pump assembly comprising at least one peristaltic pump comprising a housing with a cylindrical inner cavity with a floor and a U-shaped inner wall, at least one roller arm, an exit slot adjacent the inner wall where blood conducting tubing can leave the pump, a sensor structure in the exit slot, the sensor structure comprising an inner protrusion and an outer protrusion, and means in the sensor structure for detecting air in a tube of the tubing set when the tubing set is mounted on the centrifuge blood separation apparatus.

It is also a preferable aspect of the invention to provide, at the sensor structure, an upper chamfer adjacent a tapered surface and a vertical surface below the tapered surface.

Yet another feature of the invention may include means for mechanically and automatically drawing a blood component tube into contact with the sensor structure.

The apparatus may further include signal processing circuitry in electrical communication with the sensor structure, the signal processing circuitry being sealed in a recess in the housing.

According to the invention, a sonic pulse echo sensor is mounted in a floor between the protrusions such that a signal from the pulse sensor passes through a return loop and reflects at an interface of the return tube with surrounding air back to the pulse echo sensor.

These and other features of the invention will be apparent from the following description, together with the accompanying drawings. It is to be understood that both the foregoing general description and the following detailed description are exemplary, and are intended to provide further explanation of the invention as claimed.

The invention will now be further described by way of example, with reference to the accompanying drawings, in which:
FIG. 1 is a schematic view of one embodiment of an apheresis system, which can be used in or with the present invention.
FIG. 2 illustrates a tubing and bag set including an extracorporeal tubing circuit, a cassette assembly, and collection bag assembly, for use in or with the system of FIG. 1.

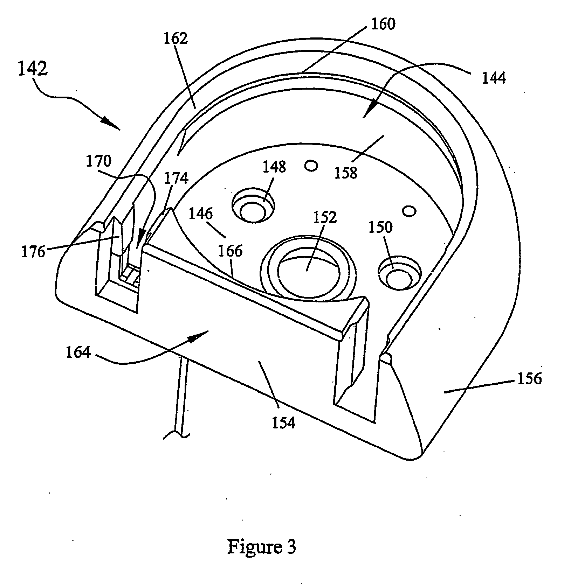

FIG. 3 is a top perspective view of peristaltic pump housing, pursuant to the present invention.

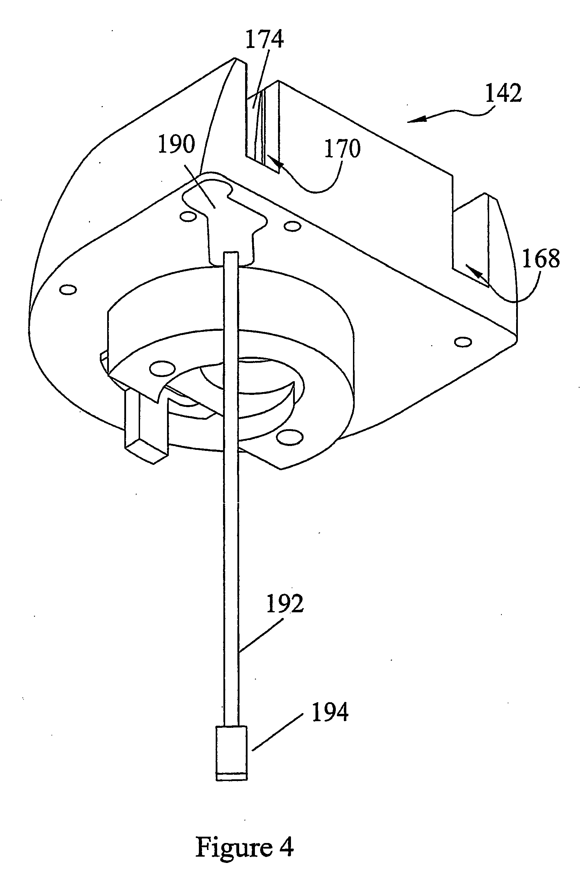

FIG. 4 is a bottom perspective view of the pump housing of Fig. 3.

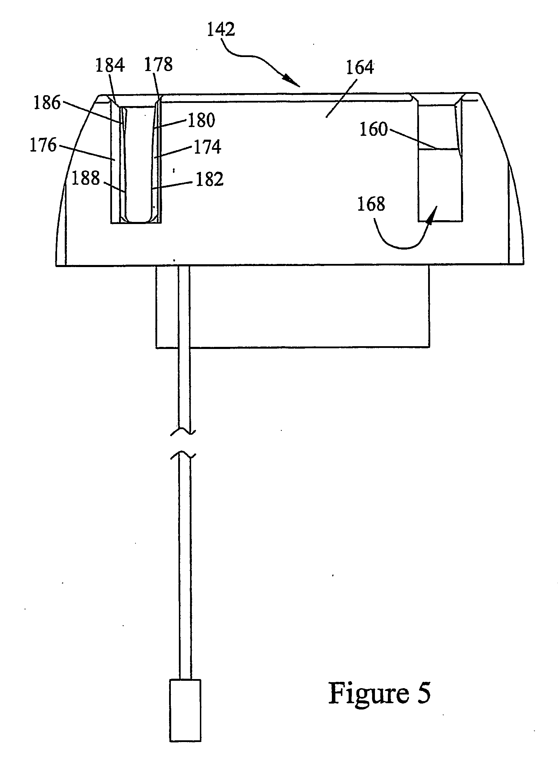

FIG. 5 is a front plan view of the pump housing of Fig. 3.

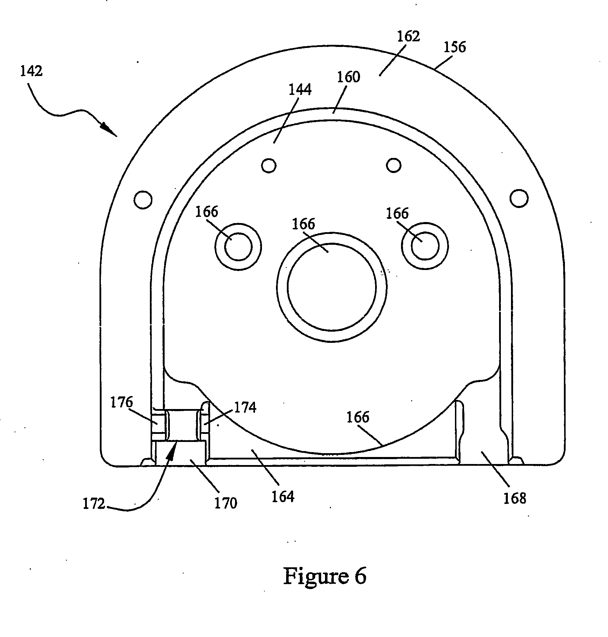

FIG. 6 is a top plan view of the pump housing of Fig. 3.
FIG. 7 is a block diagram of signal processing circuitry.

To describe the present invention, reference will now be made to the accompanying drawings. The present invention may be used with a centrifugal blood processing apparatus such as a SPECTRA OPTIA^{®} blood component centrifuge (see US Patent 7,422,693) manufactured by CaridianBCT, Inc or TRIMA^{®} or TRIMA ACCEL^{®} centrifuges. The invention may also be used with other blood component centrifuges. The Spectra Optia, Trima or Trima Accel centrifuges incorporate a one-omega/two-omega seal-less tubing connection as disclosed in U.S. Patent No. 4,425,112 to Ito, as know in the art, to provide a continuous flow of blood to and from the rotor of an operating centrifuge without requiring a rotating seal.

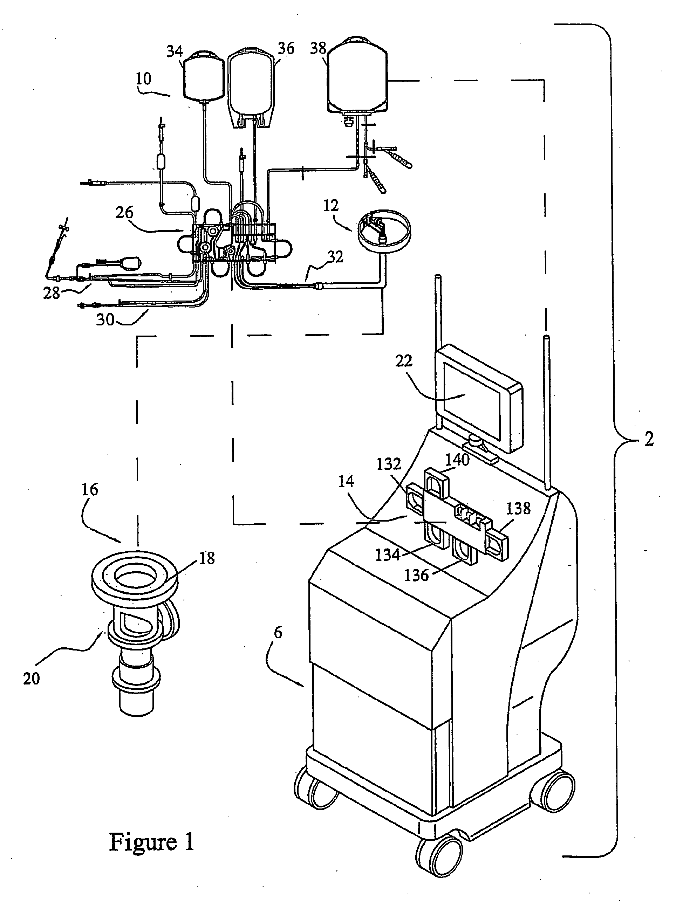

A preferred blood apheresis system 2 for use with the present invention is schematically illustrated in FIG. 1. System 2 provides for a continuous blood component separation process. Generally, whole blood is withdrawn from a donor and is substantially continuously provided to a blood component separation device 6 where the blood is separated into various components and at least one of these blood components is collected from the device 6. One or more of the separated blood components may be either collected for subsequent use or returned to the donor. In the blood apheresis system 2, blood is withdrawn from the donor and directed through a bag and tubing set 8, which includes an extracorporeal tubing circuit 10, and a blood processing vessel 12, which together define a closed, sterile and disposable system. The set 8 is adapted to be mounted in the blood component separation device 6. The separation device 6 includes a pump/valve/sensor assembly 14, which interfaces with the extracorporeal tubing circuit 10, and a centrifuge assembly 16, which interfaces with the blood processing vessel 12.

The centrifuge assembly 16 may include a channel 18 in a rotatable rotor assembly 20, which provides the centrifugal forces required to separate blood into its various blood component types by centrifugation. The blood processing vessel 12 may then be fitted within the channel 18. Blood can flow substantially continuously from the donor, through the extracorporeal tubing circuit 10, and into the rotating blood processing vessel 12. Within the blood processing vessel 12, blood may be separated into various blood component types and at least one of these blood component types (e.g., white blood cells, platelets, plasma, or red blood cells) may be removed from the blood processing vessel 12. Blood components that are not being retained for collection or for therapeutic treatment (e.g., platelets and/or plasma) are also removed from the blood processing vessel 12 and returned to the donor via the extracorporeal tubing circuit 10. Various alternative apheresis systems (not shown) may also make use of the present invention, including batch processing systems (non-continuous inflow of whole blood and/or non-continuous outflow of separated blood components) or smaller scale batch or continuous RBC/plasma separation systems, whether or not blood components may be returned to the donor.

Operation of the blood component separation device 6 is controlled by one or more processors included therein, and may advantageously comprise a plurality of embedded computer processors to accommodate interface with ever-increasing PC user facilities (e.g., CD ROM, modem, audio, networking and other capabilities). In order to assist the operator of the apheresis system 2 with various aspects of its operation, the blood component separation device 6 includes a graphical interface 22 with an interactive touch screen.

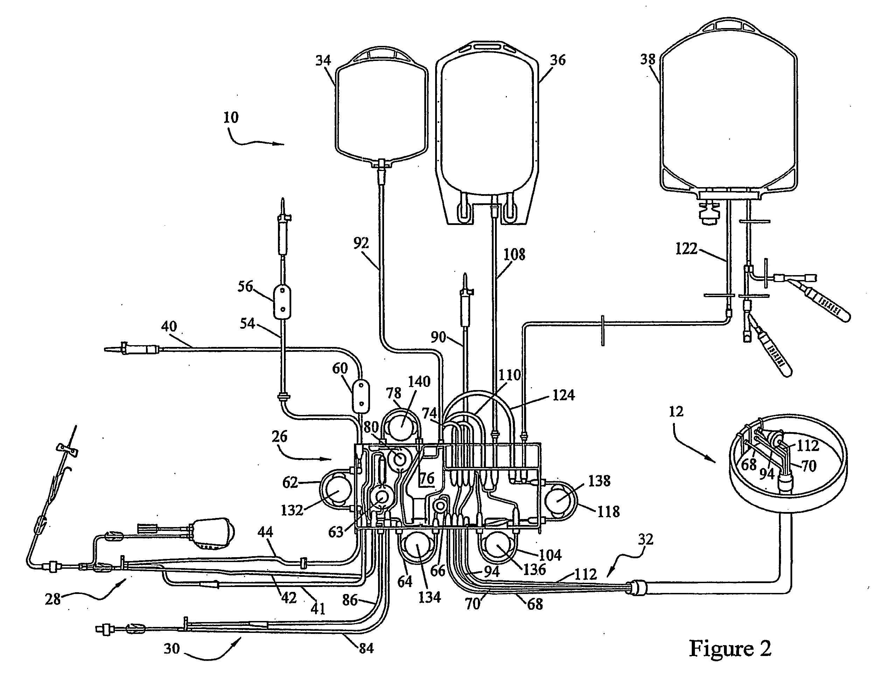

An extracorporeal tubing circuit 10, shown in FIG. 2, may include a cassette 26 and a number of tubing/collection assemblies 28, 30, 32, 34, 36, 38 and 40. A blood removal tubing assembly 28 provides a needle interface for withdrawing blood from a donor to the remainder of the tubing circuit 10. A blood return tubing assembly 30 provides a needle interface for returning blood components and other fluids to the donor. A single needle interface may also be used. Three lines 41, 42, 44 are provided in blood removal tubing assembly 28 for removal of blood from the donor. A cassette 26 is connected between the tubing assembly 28, which connects to the donor, and blood inlet/blood component tubing line sub-assembly 32, which provides the interface between cassette 26 and blood processing vessel 12. The cassette 26 orients tubing segments in predetermined spaced relationships within the cassette 26 for ultimate engagement with valve members on apheresis device 6. Such valves will, when activated, control flow through loops and tubing.

Four lines 68, 70, 94 and 112 are shown in FIG. 2 for transport of blood and components to and from the processing vessel 12. An anticoagulant tubing assembly 40, a vent bag 34, a plasma collection assembly 36, and a white blood cell collection bag 38 are also interconnected with cassette 26. The extracorporeal tubing circuit 10 and blood processing vessel 12 are pre-connected to form a closed, sterilized, disposable assembly for a single use.

When the tubing circuit 10 has been mounted on the blood component separation device 6, saline solution primes the tubing circuit through a saline line 54 and filter 56 (see FIG. 2). Saline flows through an internal passageway in the cassette 26 and through the line 41 to the distal end of the blood removal assembly 28. Saline can then flow up a blood withdrawal line 42 into the other tubes and passageways of the circuit 10 and up an anticoagulant line 44 in preparation for blood processing. A supply or bag (not shown) of anticoagulant connects to a distal end of the anticoagulant tubing assembly 40. Anticoagulant solution flows past a filter 60 and a first pump loop 62 through the anticoagulant line 44 to the distal end of the blood removal assembly. The pump loop 62 and other pump loops 64,104,118, and 78 described herein couple with peristaltic pumps 132,134,136, 138 and 140 on the blood processing device 6 in a known manner and which are shown in Fig. 2 with roller arms for forcing fluid through an adjacent tube. The housing of peristaltic pump 140 has unique features, described below, that permit it to connect a sensor to the pump loop 78 for detecting air bubbles in the tubing circuit 10 before blood or other fluids are returned to the donor. The device 6 controls the direction and rate of flow of the fluids described herein by controlling the speed and direction of the peristaltic pumps and the position of various valves.

The blood removal line 42 conducts blood into the cassette 26, where the blood passes a first pressure sensor 63 and a second pump loop 64. A second pressure sensor 66, between second pump loop 64 with its associated pump 134 and blood inflow line 68 to the blood processing vessel 12, senses the fluid pressure effective at an inlet to the blood processing vessel 12. Emanating from blood processing vessel 12 is an RBC outlet tubing line 70 of the blood inlet/blood component tubing assembly 32. The outlet tubing line 70 connects to an external loop 74 to a return reservoir 76. The return reservoir 76 contacts sensors on the device 6 that detect low and high fluid levels. The device 6 keeps the fluid in the reservoir between these two levels by controlling flow out of the reservoir past a return pump loop 78, which is coupled to the return pump 140, and a return pressure sensor 80. As the fluid level in the reservoir 76 is constantly rising and falling, a vent bag 34 connects to the reservoir 76 through a vent tube 92. Air can flow between the reservoir 76 and the vent bag 34 in a sterile manner. Fluid flows into a return tube 84 in the blood return assembly 30. The return assembly 30 also comprises a saline line 86 connected internally in the cassette 26 to saline line 54 for priming as described above. If desired, red blood cells could be withdrawn through the replacement line 90 and collected in a collection bag (not shown).

Plasma may also be collected from the blood processing vessel 12 into plasma bag 36. When desired, plasma is withdrawn from the blood processing vessel 12 through plasma line 94 to a pump loop 104, which is coupled to a pump 136. A valve (not shown) diverts the plasma either into a collect tube 108 to the plasma bag 36, or into a connecting loop 110 to the reservoir 76. Excess plasma in the reservoir 76 is returned to the donor in the same way as red blood cells, as described above.

White blood cells flow out of the blood processing vessel 12 through a fourth cell line 112 in the tubing line sub-assembly 32. In the cassette 26, a red-green photo sensor (not shown) may be used to control periodic flushing of white blood cells out of the blood processing vessel 12 into the collect bag 38. The white blood cells flow through a pump loop 118, which engages a peristaltic pump 138 on the separation device 6. The pump loop 118 connects to a valved passageway in the cassette 26. The blood processing device 6 can control a valve to direct white blood cells either into a collect tube 122 and thence into the collect bag 38, or into a connection loop 124 and thence into the reservoir 76. Excess white blood cells in the reservoir 76 may be returned to the donor in the same way as red blood cells and plasma, as described above.

During a blood removal, whole blood will be passed from a donor into tubing line 42 of blood removal tubing assembly 28. The blood is pumped by the device 6 via pump loop 64, to the blood processing vessel 12 via the cassette 26 and line 68 of the blood inlet/blood component tubing assembly 32. Separation processing then occurs on a substantially continuous basis in the blood processing vessel 12, i.e., blood flows substantially continuously therein, is continuously separated and flows as separated components therefrom. After separation processing in vessel 12 (though separation is continuously occurring), uncollected blood components are transferred from the processing vessel 12 to and through cassette 26, into reservoir 76 of cassette 26 up to a predetermined level. The blood component separation device 6 may initiate a blood return submode wherein components may be returned to the donor through return line 84. The cycle between blood removal and blood return submodes will continue until a predetermined amount of blood components have been harvested. In an alternative single needle scheme, as is known in the art, blood may be alternately removed from the donor and returned to a donor through a single needle.

In the process of removing blood from a donor, separating the blood into components, adding anticoagulant or replacement fluids, and returning blood components to a donor, air bubbles may be inadvertently introduced into the fluid. Small air bubbles are of little or no consequence, but large air bubbles in the returned blood components may be painful or harmful to the donor. It is desirable to monitor the returning blood components for air bubbles that are larger than a predetermined size. The apparatus of this invention provides a sensor in the outflow race of the return peristaltic pump 140. The structure of the housing allows the return loop 78 to be brought reliably into contact with the sensor automatically as the cassette 26 is mounted on the blood component separation device 6.

The peristaltic pump 140 comprises a housing 142. The housing 142 comprises a cylindrical inner cavity 144 with a floor 146. The floor 146 has two countersunk bores 148, 150 for machine screws (not shown) to mount the housing on the separation device 6. A central opening 152 allows a shaft to drive roller arms (shown in Fig. 2) of the peristaltic pump in a known fashion. See, for instance, US patent 5,263,831. The housing 142 has a planar outer wall 154 which is configured to abut the cassette 26. A U-shaped outer wall 156 completes the outer shape of the housing. The cavity 144 has a U-shaped inner wall 158 against which the return loop 78 rests when the loop 78 is mounted within the peristaltic pump 140. A ridge 160 may be provided along an upper edge 162 of the inner wall 158 to secure the return loop 78 within the housing when the pump is compressing loop by action of the roller arms. The planar outer wall 154 comprises a central section 164 having an arcuate surface 166 that is congruent with the inner radius of the return loop 78. The arcuate surface 166 helps the roller arms to act with uniform force on the return loop 78 as the arms rotate within the housing.

An entrance slot 168 in the planar outer wall 154 allows the return loop 78 to enter the inner cavity 144 of the housing 142, where the loop 78 lies along the U-shaped inner wall 158. The loop 78 leaves the housing through an exit slot 170 in the planar wall 154. A sensor structure 172 is uniquely mounted in the exit slot 170 for detecting air bubbles in the fluid within the return loop 78.

The sensor structure 172 comprises an inner protrusion 174 from the central section 164. The inner protrusion faces a similar outer protrusion 176 on the inner wall 158. Both protrusions 174, 176 are located in the exit slot 170. The inner protrusion 174 has an upper chamfer 178 adjacent a tapered surface 180. Below the tapered surface 180 is a vertical surface 182. Facing the upper chamfer 178, tapered surface 180 and vertical surface 182 of the inner protrusion 174, the outer protrusion 176 also has an upper chamfer 184, a tapered surface 186, and a vertical surface 188. When the bag and tubing set 8 is mounted on the blood processing device 6, the cassette 26 is mechanically and automatically drawn into place on the device 6, as known in the art. In particular, the return loop 78 is drawn into the peristaltic pump 140. Usually such a loop only contacts the U-shaped inner wall 158 and the roller arms of the peristaltic pump. This presents very little frictional resistance to mounting the tubing set 8. In this invention, however, the return loop 78 is also brought into close contact with the inner and outer protrusions 174, 176 of the sensor structure 172. Because the return loop 78 is attached to the rigid cassette 26, the act of mounting the cassette 26 on the device 6 also wedges a portion of the return loop 78 between the protrusions 174, 176. The portion of the return loop 78 between the protrusions 174, 176 will be deformed vertically into a generally elliptical shape, with outer surfaces in contact with an appropriate sensor in the protrusions. This structure allows the blood component tube to be mechanically and automatically drawn into contact with the sensor. In an unclaimed embodiment, such a sensor may be a sonic sensor such as are available from Moog Medical Devices Group (formerly known as Zevex Applied Technology), Salt Lake City, Utah, for example. A sonic transmitter may be mounted in one protrusion, for example the outer protrusion 176, and a receiver may be mounted in the other protrusion, for example the inner protrusion 174. Signal processing circuitry for the sensor may be sealed in a recess 190 in the housing 142, and a cable 192 and connector 194 may be provided for coupling the sensor to control circuitry, such a microprocessor, in the device 6.

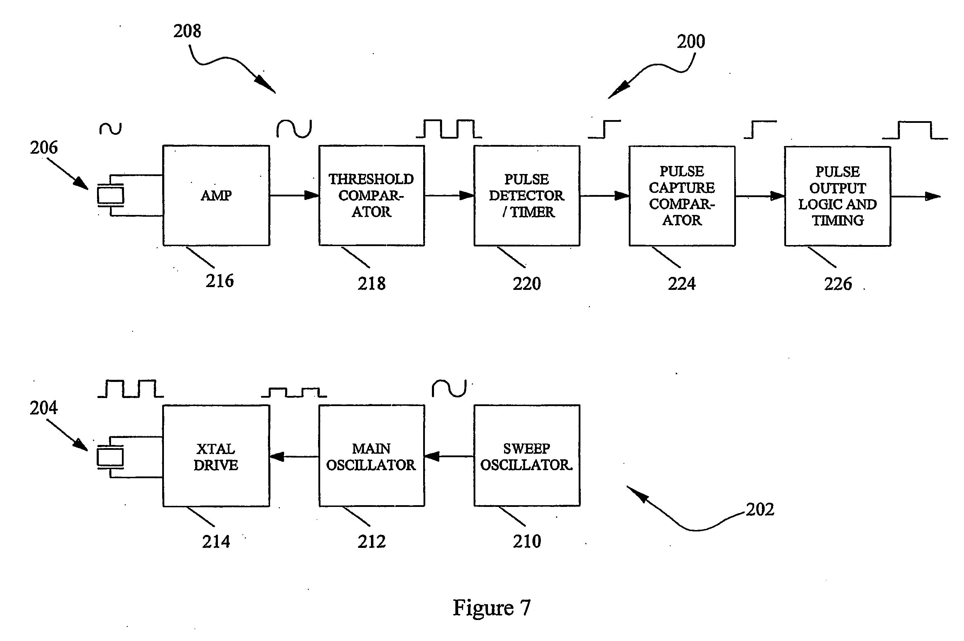

Figure 7 shows a block diagram of signal processing circuitry 200 for the sensor. The circuitry 200 comprises a transmitter 202 that drives an emitting crystal 204 and a receiver 208 that responds to a receiving crystal 206. The emitting crystal 204 produces a sonic output that is propagated through an adjacent tube and fluid to the receiving crystal, which transforms the mechanical sonic waves into an electrical signal. The propagation of the sonic waves is impeded by air bubbles in the tube, allowing the bubbles to be detected. The transmitter 202 is operational when power is supplied to it, usually whenever the apheresis system 2 is active. However, the transmitter may also be configured to interrupt its output for test purposes in response to a signal from a controller or microprocessor (not shown). Such an interruption of the transmitter signal would produce an output from the receiver 208 equivalent to sensing an air bubble in the adjacent tube. A sweep modulation oscillator 210 produces a sweep signal that varies the frequency of a wave produced by a main oscillator 212. This spreads the energy of the main oscillator signal over a certain frequency range, thereby avoiding interference with other devices, as regulatory authorities such as the FCC. The signal is delivered by a crystal driver 214 to the emitting crystal 204 at a proper amplitude to cause the crystal to emit a sonic signal.

The sonic signal passes through an adjacent tube and fluid to the receiving crystal 206. If there are bubbles present in the tube, the signal will be weakened or lost and will not produce a response in the receiving crystal 206 to be detected by the receiver 208. The output of the receiving crystal 206 is delivered to an amplifier 216 before being processed by a threshold comparator circuit 218. The analog output of crystal 206 is first amplified by the differential amplifier 216 to improve sensitivity to the fluid-coupled signal and to improve the signal-to-noise ratio. Then the signal is compared to a selected threshold voltage in the threshold comparator 218, which converts the signal to a pulse train output of equivalent frequency. Only if the amplified output of the crystal 206 exceeds the threshold is a pulse train signal sent to a missing pulse detector/timer circuit 220. If there is fluid in the tube, a pulse train signal will be presented to the pulse detector circuit 220, which will continue to re-set itself and produce a null output as long as the pulse train signal is present. If bubbles appear in the tube and cause the pulse train signal to the pulse detector circuit 220 to be interrupted for a selected period of time, the pulse detector circuit 220 produces an output pulse. The output pulse is communicated to a pulse capture comparator 224 which standardizes the output of the pulse detector circuit 220 to a preselected trigger signal. A pulse output logic and timing circuit 226 responds to the standardized output or trigger signal of the pulse capture comparator 224 by producing a consistent minimum duration logic pulse of selected duration that can be recognized by the microprocessor or controller as an indication that a significant bubble has been detected. The pulse output logic circuit 226 may be configured produce a consistent minimum time logic pulse of a selected duration, even if bubble size, number or velocity in fluid flow are of shorter duration than the desired pulse width output. The signal processing circuitry, therefore, always produces a digital output signal of preselected minimum duration to the controller even if a small or fast-moving volume of air has been detected in the adjacent tube.

As an alternative to the sonic transmitter and receiver and according to the invention, a sonic pulse echo sensor is employed. Such a sensor is mounted in the floor 146 such that the signal from the sensor would pass through the return loop 78 and any fluid contained therein and would be reflected at the interface of the return tube with surrounding air back to the pulse echo sensor.

It has been found that a sonic sensor, as described above, can detect a wide range of sizes of air bubbles, many of which are so small that they pose no threat to either the health or comfort of the donor. It is important, therefore to select a pre-determined minimum bubble size or sizes or a cumulative volume, and to warn the device operator only of the existence of bubbles that exceed a certain size or of a cumulative volume of bubbles, or to stop the blood donation procedure if a bubble exceeds a certain critical size or if a pre-determined volume of bubbles over a certain period or volume of fluid. Other actions responsive to a detected condition might be initiating manual or automatic recovery procedures, such as reversing the peristaltic pump to return fluid and gas to the reservoir 78. To detect an error condition, the apparatus may use computer control to add together the volumes of a plurality of bubbles. A running total (first in, first out) of the sum of bubble volumes over either a period of time or a selected volume of fluid could be used to detect an alarm condition. An internal clock in the on-board computer could provide time measurement. Fluid volume could also be calculated by the computer as a function of a number of revolutions of the peristaltic pump.

Currently, it is considered desirable for the system to detect 0.060 mL bubbles at a flow rate of 295 mL/min. This would correlate to 1 cm of air in a 2.8 mm ID tube. This specification is very tight and while it does address the perception of "too much air" in the return line, it does not reflect the actual safety limits on air in the return line. A specification of less than 1 mL of air detected at the same flow rate is more appropriate. Since the peristaltic pump will tend to cut up a bubble into smaller segments, the apparatus will need to add up air over some time period and alarm if the additive air reaches 1 mL over a selected period of time.

Air bubble detection sensors could also be placed at other locations within the return pump 140 or in the cassette 26 downstream from the return reservoir.

It is desirable to avoid false alarms, that is, false positive indications of air bubbles. Acceptable performance may be defined in terms of number of allowed false alarms per 1000 runs or some other probability of occurrence.

On detection of a bubble of sufficient size, duration or cumulative volume, the operator should have the ability to clear an alarm, if appropriate. In some instances, air in the return line may be caused from clotting off the lower level sensor in the return reservoir 76, whereupon air would be drawn into the system. This is usually considered to be a non-recoverable condition. Other causes for air detections (possibly false detections) may be recoverable. The operator can decide when clearing the bubble is non-productive and choose to end the run at that time. There will be limits, however, on the number of time the operator can clear the air, based on physical constraints of the system. Constraints include vent bag 34 volume, or the reservoir 76 upper level sensor (volume in reservoir), among other possible limits.

There should not be a complete override of the air detector. Otherwise, the operator may wrongly assume that since the system is allowing the procedure to continue, it is safe to do so without checking the conditions that raised the alarm. The purpose of the return line bubble detector is to serve as a redundant sensor to the pre-existing monitoring of the lower level sensor.
Its major purpose is to eliminate most of the sources of error that could raise a LLS alarm and confirm that air is the cause of the alarm.

The following procedure could be used to respond to a bubble detection alarm. An alarm is raised for air detected in return line 78. The operator is instructed to clamp the return needle. Software checks for clamp closure (pressure check). The operator is instructed to open return saline roller clamp. The return pump 140 pumps a fixed quantity of saline through return line to clear bubble. The pump stops when or shortly after the bubble detector again sees fluid. If the bubble detector does not detect fluid during recovery, or if the upper level sensor in the return reservoir 76 is reached, or if the return pump has pumped more than 350 mL cumulative over all air recovery operations (that is, the vent bag volume), then air recovery stops and the run ends without rinseback. All air detection events will require recovery until vent bag volume is reached or upper level sensor in the reservoir 76 detects fluid.

If ending the run without rinseback becomes the only option, the operator is instructed to close a saline line roller clamp. Software checks for closure of roller clamp (pressure check). The operator is instructed to open return line clamp. The operator confirms and restarts the procedure.

This description is not to be construed as a limitation on the scope of the invention. It will be apparent to those skilled in the art that various modifications and variations can be made to the structure and methodology of the present invention without departing from the scope of the invention as defined in the claims.

## Claims

1. A peristaltic pump (140) comprising:
a housing (142) with a cylindrical inner cavity (144) with a floor (146) and a U-shaped inner wall (158),
at least one roller arm,
an exit slot (170) adjacent said inner wall where blood conducting tubing can leave the pump,
sensor structure (172) in said exit slot, said sensor structure (172) comprising an inner protrusion (174) and an outer protrusion (176), and
means in said sensor structure for detecting air in an adjacent tube,
**characterised in that**:
said means for detecting air comprises a sonic pulse echo sensor,
said sonic pulse echo sensor is mounted in a floor (146) between said protrusions such that a signal from the pulse sensor passes through a return loop and reflects at an interface of the return tube with surrounding air back to the pulse echo sensor.

2. The peristaltic pump of claim 1 wherein said sensor structure further comprises at least one upper chamfer (178) adjacent a tapered surface (180) and a vertical surface (182) below the tapered surface.

3. The peristaltic pump of claim 1, further comprising means (14) for mechanically and automatically drawing a blood component tube into contact with said sensor structure.

4. The peristaltic pump of claim 1, further comprising signal processing circuitry (200) in electrical communication with the sensor structure, said signal processing circuitry being sealed in a recess (190) in the housing.

5. A centrifuge blood separation apparatus (2) comprising:
a rotor assembly (20),
a pump assembly adapted to interface with a tubing set, said pump assembly comprising:
at least one peristaltic pump (140) according to any one of the preceding claims;
wherein said means in said sensor structure for detecting air in an adjacent tube is for detecting air in a tube of said tubing set mounted on said centrifuge blood separation apparatus (2).

6. A method of detecting air bubbles in a tube of a tubing set (10) on a centrifuge blood separation apparatus (2) comprising a rotor assembly (20) and a pump assembly adapted to interface with said tubing set, said method comprising:
providing a sensor structure (172) in an exit slot of at least one peristaltic pump,
placing the tube of said tubing set in said peristaltic pump adjacent said sensor structure, and
detecting air in said tube of said tubing set with said sensor structure,
**characterised by**:
said sensor structure comprising a sonic pulse echo sensor, wherein said sonic pulse echo sensor is mounted in a floor between protrusions of said sensor structure such that a signal from the pulse echo sensor passes through a return loop and reflects at an interface of the return tube with surrounding air back to the pulse echo sensor.

7. The method of claim 6, wherein said sensor structure further comprises:
at least one upper chamfer (178) adjacent a tapered surface (180) and a vertical surface (182) below the tapered surface.

8. The method of claim 6, further comprising mechanically and automatically drawing a blood component tube into contact with the sensor structure (172).

9. The method of claim 6, further comprising mounting signal processing circuitry in electrical communication with the sensor structure, said signal processing circuitry being sealed in a recess in the housing.

## Patentansprüche

1. Peristaltische Pumpe (140), die Folgendes umfasst:
ein Gehäuse (142) mit einem zylindrischen inneren Hohlraum (144) mit einem Boden (146) und einer U-förmigen Innenwand (158),
mindestens einen Rollenarm,
einen Ausgangsschlitz (170) benachbart zu der Innenwand, wo blutleitende Schläuche aus der Pumpe austreten können,
eine Sensorstruktur (172) in dem Ausgangsschlitz, wobei die Sensorstruktur (172) einen inneren Vorsprung (174) und einen äußeren Vorsprung (176) umfasst und
ein Mittel in der Sensorstruktur zur Erfassung von Luft in einem benachbarten Schlauch,
**dadurch gekennzeichnet, dass**:
das Mittel zum Erfassen von Luft einen Schallimpuls-Echosensor umfasst,
wobei der Schallimpuls-Echosensor in einem Boden (146) zwischen den Vorsprüngen angebracht ist, so dass ein Signal von dem Impulssensor eine Rücklaufschleife durchläuft und an einer Grenzfläche des Rücklaufschlauchs mit umgebender Luft zu dem Impuls-Echosensor zurück reflektiert wird.

2. Peristaltische Pumpe nach Anspruch 1, wobei die Sensorstruktur ferner Folgendes umfasst: mindestens eine obere Abschrägung (178) benachbart zu einer sich verjüngenden Fläche (180) sowie eine vertikale Fläche (182) unterhalb der abgeschrägten Fläche.

3. Peristaltische Pumpe nach Anspruch 1, die ferner ein Mittel (14) zum mechanischen und automatischen Ziehen eines Blutkomponentenschlauchs in Kontakt mit der Sensorstruktur umfasst.

4. Peristaltische Pumpe nach Anspruch 1, die ferner eine Signalverarbeitungsschaltung (200) in elektrischer Kommunikation mit der Sensorstruktur umfasst, wobei die Signalverarbeitungsschaltung in einer Vertiefung (190) in dem Gehäuse abgedichtet ist.

5. Blutzentrifugentrennvorrichtung (2), die Folgendes umfasst:
eine Rotorbaugruppe (20),
eine Pumpenbaugruppe, die dafür ausgelegt ist, mit einem Schlauchsatz verbunden zu werden, wobei die Pumpenbaugruppe Folgendes umfasst:
mindestens eine peristaltische Pumpe (140) nach einem der vorhergehenden Ansprüche;
wobei das Mittel in der Sensorstruktur zur Erfassung von Luft in einem benachbarten Schlauch dazu dient, Luft in einem Schlauch des Schlauchsatzes zu erfassen, der an der Blutzentrifugentrennvorrichtung (2) angebracht ist.

6. Verfahren zum Erfassen von Luftblasen in einem Schlauch eines Schlauchsatzes (10) an einer Blutzentrifugentrennvorrichtung (2), die Folgendes umfasst:
eine Rotorbaugruppe (20) und eine Pumpenbaugruppe, die dafür ausgelegt ist, mit dem Schlauchsatz verbunden zu werden, wobei das Verfahren Folgendes umfasst:
Bereitstellen einer Sensorstruktur (172) in einem Ausgangsschlitz von mindestens einer peristaltischen Pumpe,
Anordnen des Schlauchs des Schlauchsatzes in der peristaltischen Pumpe benachbart zu der Sensorstruktur, und
Erfassen von Luft in dem Schlauch des Schlauchsatzes mit Hilfe der Sensorstruktur,
**dadurch gekennzeichnet dass**:
die Sensorstruktur einen Schallimpuls-Echosensor umfasst, wobei der Schallimpuls-Echosensor in einem Boden zwischen Vorsprüngen der Sensorstruktur angebracht ist, so dass ein Signal von dem Impuls-Echosensor eine Rücklaufschleife durchläuft und an einer Grenzfläche des Rücklaufschlauchs mit umgebender Luft zu dem Impuls-Echosensor zurück reflektiert wird.

7. Verfahren nach Anspruch 6, wobei die Sensorstruktur ferner Folgendes umfasst:
mindestens eine obere Abschrägung (178) benachbart zur einer sich verjüngenden Fläche (180) sowie eine vertikale Fläche (182) unterhalb der abgeschrägten Fläche.

8. Verfahren nach Anspruch 6, das ferner das mechanische und automatische Ziehen eines Blutkomponentenschlauchs in Kontakt mit der Sensorstruktur umfasst.

9. Verfahren nach Anspruch 6, das ferner die Anbringung einer Signalverarbeitungsschaltung in elektrischer Kommunikation mit der Sensorstruktur umfasst, wobei die Signalverarbeitungsschaltung in einer Vertiefung in dem Gehäuse abgedichtet ist.

## Revendications

1. Pompe péristaltique (140) comprenant :
un logement (142) avec une cavité interne cylindrique (144) avec un plancher (146) et une paroi interne en U (158),
au moins un bras à rouleau,
une fente de sortie (170) adjacente à ladite paroi interne où la tubulure de conduite de sang peut quitter la pompe,
une structure de capteur (172) dans ladite fente de sortie, ladite structure de capteur (172) comprenant une protubérance interne (174) et une protubérance externe (176), et
un moyen dans ladite structure de capteur pour détecter de l'air dans un tube adjacent,
**caractérisée en ce que** :
ledit moyen pour détecter de l'air comprend un capteur à ultrasons,
ledit capteur à ultrasons est monté dans un plancher (146) entre lesdites protubérances de sorte qu'un signal provenant du capteur à ultrasons passe à travers une boucle de retour et soit renvoyé au niveau d'une interface du tube de retour avec l'air environnant vers le capteur à ultrasons.

2. Pompe péristaltique de la revendication 1, dans laquelle ladite structure de capteur comprend en outre au moins un chanfrein supérieur (178) adjacent à une surface évasée (180) et une surface verticale (182) sous la surface évasée (180).

3. Pompe péristaltique de la revendication 1, comprenant en outre un moyen (14) pour attirer mécaniquement et automatiquement un tube de composant sanguin pour le mettre en contact avec ladite structure de capteur.

4. Pompe péristaltique de la revendication 1, comprenant en outre des circuits de traitement de signaux (200) en communication électrique avec la structure de capteur, lesdits circuits de traitement de signaux étant scellés dans un évidement (190) dans le logement.

5. Appareil de séparation centrifuge du sang (2) comprenant :
un ensemble de rotor (20),
un ensemble de pompe adapté pour former une interface avec le jeu de tubulure, ledit ensemble de pompe comprenant :
au moins une pompe péristaltique (140) selon l'une quelconque des revendications précédentes ;
dans lequel ledit moyen dans ladite structure de capteur pour détecter de l'air dans un tube adjacent est destiné à détecter de l'air dans un tube dudit jeu de tubulure monté sur ledit appareil de séparation centrifuge du sang (2).

6. Procédé de détection de bulles d'air dans un tube d'un jeu de tubulure (10) sur un appareil de séparation centrifuge du sang (2) comprenant un ensemble de rotor (20) et un ensemble de pompe adapté pour former une interface avec ledit jeu de tubulure, ledit procédé comprenant :
la fourniture d'une structure de capteur (172) dans une fente de sortie d'au moins une pompe péristaltique,
le placement du tube dudit jeu de tubulure dans ladite pompe péristaltique adjacent à ladite structure de capteur, et
la détection de l'air dans ledit tube dudit jeu de tubulure avec ladite structure de capteur,
**caractérisé en ce que** :
ladite structure de capteur comprend un capteur à ultrasons, ledit capteur à ultrasons étant monté dans un plancher entre des protubérances de ladite structure de capteur de sorte qu'un signal provenant du capteur à ultrasons passe à travers une boucle de retour et soit renvoyé au niveau d'une interface du tube de retour avec l'air environnant vers le capteur à ultrasons.

7. Procédé selon la revendication 6, dans lequel ladite structure de capteur comprend en outre :
au moins un chanfrein supérieur (178) adjacent à une surface évasée (180) et une surface verticale (182) sous la surface évasée.

8. Procédé selon la revendication 6, comprenant en outre le fait d'attirer mécaniquement et automatiquement un tube de composant sanguin pour le mettre en contact avec la structure de capteur (172).

9. Procédé selon la revendication 6, comprenant en outre le montage de circuits de traitement de signaux en communication électrique avec la structure de capteur, lesdits circuits de traitement de signaux étant scellés dans un évidement dans le logement.
